**Europäisches Patentamt**

**European Patent Office**

**Office européen des brevets**

(19)

(11) Numéro de publication: **0 079 844**
**B1**

# (12) FASCICULE DE BREVET EUROPEEN

(45) Date de publication du fascicule du brevet:
**19.02.86**

(21) Numéro de dépôt: **82420149.5**

(22) Date de dépôt: **25.10.82**

(51) Int. Cl.⁴: **A 61 F 2/24**

(54) **Prothèse valvulaire cardiaque.**

(30) Priorité: **05.11.81 FR 8120898**

(43) Date de publication de la demande:
**25.05.83 Bulletin 83/21**

(45) Mention de la délivrance du brevet:
**19.02.86 Bulletin 86/8**

(84) Etats contractants désignés:
**AT BE CH DE FR GB IT LI LU NL SE**

(56) Documents cités:
**AU - B - 450 689**
**FR - A - 1 503 297**
**FR - A - 1 503 297**
**US - A - 3 579 642**
**US - A - 3 938 197**
**US - A - 4 274 437**

(73) Titulaire: **Murguet, Robert Félicien, 4 rue de Constantine,**
**F-69001 Lyon (Rhône) (FR)**
Titulaire: **Tardy, épouse Lugarini, Joelle Marie-Thérèse**
**Jeanne, 11 Résidence du Val, Palaiseau (Essonne) (FR)**

(72) Inventeur: **Murguet, Robert Félicien, 4 rue de**
**Constantine, F-69001 Lyon (Rhône) (FR)**
Inventeur: **Tardy, épouse Lugarini, Joelle Marie-Thérèse**
**Jeanne, 11 Résidence du Val, Palaiseau (Essonne) (FR)**

(74) Mandataire: **Maureau, Philippe, Cabinet Germain &**
**Maureau Le Britannia - Tour C 20, bld Eugène Déruelle,**
**F-69003 Lyon (FR)**

## Description

La présente invention a pour objet une prothèse valvulaire cardiaque pour le remplacement des valves cardiaques lésées.

Si, depuis quelques années, la science chirurgicale apporte, par implantation des prothèses valvulaires, de grands services procurant une survie appréciable aux malades atteints de cardiopathie valvulaire, il n'en demeure pas moins que le fonctionnement et la fiabilité des prothèses sont soumis aux effets secondaires de certains impératifs hydrodynamiques, tels que formation thromboembolique et agressions hémolytiques.

Actuellement, dans le cas d'un remplacement valvulaire, le chirurgien e le choix entre l'implantation d'une valve biologique ou d'une valve mécanique.

Les valves biologiques ou bioprothèses sont confectionnées à partir de tissus animaux (hétérogreffes) ou humains (homogreffes), neutralisés, et reproduisent à peu près le mécanisme des valves naturelles. Si ces valves limitent les risques de thromboses et ne nécessitent théoriquement pas de traitement anticoagulants, leur fiabilité demeure néanmoins très incertaine.

Les valves mécaniques sont fabriquées industriellement et sont en général de deux sortes, à savoir les valves à billes et les valves à disques.

Une valve à bille est constituée par une bille montée mobile dans une cage métallique entre les positions d'ouverture et de fermeture. Ce système présente l'avantage d'une grande fiabilité, mais provoque des risques importants de thrombose, nécessitant au patient un traitement anticoagulant à vie, provoque des turbulences dans l'écoulement du sang, et est d'un fonctionnement bruyant.

Il est connu de réaliser une valve à disque comprenant une partie en forme de collerette circulaire qui, fixée sur la paroi cardiaque, délimite un passage susceptible d'être obturé par un disque en une ou deux parties, articulé sur la collerette par un système de charnières, autour d'un axe contenu dans le plan de la collerette. Dans la mesure où la prothèse comporte un disque constitué par deux volets, chacun de ceux-ci est articulé autour d'un axe situé à proximité de son extrémité la plus éloignée de son bord de contact avec l'autre volet.

De telles prothèses sont décrites dans le brevet français 1 503 297, pour ce qui concerne une valvule à deux volets, et dans le brevet australien 450 689 pour ce qui concerne une valvule à un volet.

Ce type de valvule possède de bonnes performances hémodynamiques, malgré l'existence de zones tourbillonnaires et d'effets secondaires qui exposent le patient aux mêmes risques thromboemboliques.

Il a notamment été constaté que l'écoulement du sang était particulièrement turbulent et générateur de thrombose et de dégradations hémolytiques dans les dix premiers degrés d'ouverture du clapet.

La présente invention vis à remédier à ces inconvénients.

A cet effet, dans la valvule qu'elle concerne, du type comprenant une collerette ménageant un passage susceptible d'être obturé par un disque formé de deux volets articulés sur elle et comprenant deux bords venant en contact l'un de l'autre dans la region centrale du passage, en position fermée de celui-ci, chaque volet étant articulé sur la collerette autour d'un axe situé à proximité de son extrémité la plus éloignée de son bord de contact avec l'autre volet, les deux volets sont de surfaces inégales. Compte tenu des différences de surface des deux volets, il se produit en décalage dans les ouvertures respectives de ceux-ci.

L'intensité des turbulences d'ouverture du premier volet est mise à profit pour provoquer une ouverture spontanée et rapide du second volet. Le flot sanguin peut donc s'écouler très rapidement et librement dans une zone parfaitement homogène.

Ce type de prothèse valvulaire convient tout aussi bien pour le remplacement des valves mitrales que des valves aortiques.

Avantageusement, les axes d'articulation des deux volets sont décalés, l'un par rapport à l'autre dans le sens d'écoulement du flot sanguin, l'axe du volet de plus grande surface étant situé en amont de l'axe de l'autre volet.

Selon une autre caractéristique, les extrémités des deux volets situées du côté du centre du disque et venant en contact pour réaliser la fermeture de la valve sont courbées parallèlement aux axes de rotation des volets, dans la direction d'écoulement du flot sanguin.

Lors de l'arrivée du flot sanguin, le volet de plus grande surface et dont l'axe de rotation est situé en amont commence à s'ouvrir, provoquant un écoulement tourbillonnaire qui, agissant sur la partie courbée du second volet, provoque l'ouverture spontanée et rapide de celui-ci, limitant au maximum la durée pendant laquelle l'écoulement de sang est perturbé.

En fin de passage du sang, la pression inverse referme instantanéments, sans bruit et sans heurt, les deux clapets, dont l'appui des bords respectifs l'un sur l'autre assure une fermeture étanche.

Selon une autre caractéristique de cette prothèse valvulaire, chaque volet présente une courbure perpendiculairement à son axe de rotation dans la direction d'écoulement du flot sanguin.

La combinaison de cette courbure avec celle définie précédemment est particulièrement intéressante dans le cas de prothèses valvulaires aortiques, car permettant aux volets d'être situés le plus près possible de la paroi aortique et évitant ainsi les écoulements turbulents.

Avantageusement, la collerette présente un épaulement formant siège pour les volets, lorsque ceux-ci sont en position fermée, ce qui procure une excellente étanchéité en absence d'écoulement du flot sanguin. La collerette est également conformée pour ménager des butées en position ouverte des volets.

De toute façon, l'invention sera bien comprise à l'aide de la description qui suit en référence au dessin schématique annexé représentant, à titre d'exemples non limitatifs, plusieurs formes d'exécution de cette prothèse valvulaire.

Figure 1 est une vue en perspective de cette valve en position fermée;

Figure 3 est une vue en coupe transversale en position fermée, selon la ligne 3–3 de figure 2;

Figures 4 et 5 en sont deux vues en perspective, respectivement, au cours de l'ouverture et après ouverture;

Figure 6 en est une vue en plan en position ouverte;

Figure 7 à 11 sont des vues d'une seconde forme d'exécution de cette prothèse valvulaire, correspondant, respectivement, aux vues de figures 2 à 6;

Figures 12 et 13 sont deux vues très schématiques en coupe et en position fermée d'une variante d'exécution de cette prothèse;

Figures 14 à 16 sont trois vues de l'écoulement de sang dans un tube équipé, respectivement, d'une valve à disque traiditionnelle, d'une valve à disque conforme à celle des figures 1 à 6 et d'une valve à disque conforme à celle des figures 7 à 11.

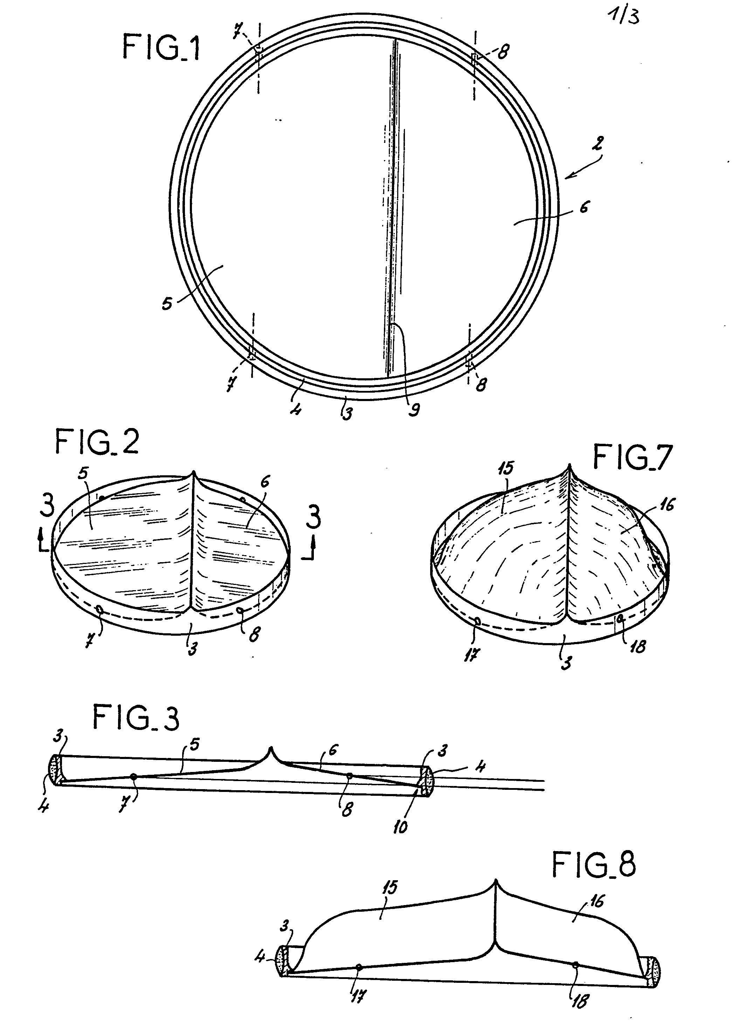

La valve (2) représentée aux figures 1 à 6, comprend une collerette circulaire (3), destinée à être fixée sur la paroi cardiaque par couture d'une garniture (4), telle qu'en polytétrafluoréthylène, qui l'entoure. Cette collerette (3) ménage un passage susceptible d'être obturé par deux volets, respectivement (5) et (6), complémentaire l'un de l'autre.

Comme il ressort clairement du dessin, la surface du volet (5) est sensiblement supérieure à celle du volet (6). Les deux volets (5) et (6) sont articulés, respectivement, autour d'un axe (7) et autour d'un axe (8), parallèles à leur ligne de contact (9). Comme montré au dessin, les deux axes (7) et (8) ne sont pas situés dans le même plan, l'axe (7) du volet (5) étant disposé en amont de l'axe (8) du volet (6), dans le sens d'écoulement du sang.

En outre, les deux volets présentent, dans leur zone située du côté de leur ligne de contact (9), une courbure parallèlement à leurs axes d'articulation respectifs (7) et (8), dans le sens d'écoulement du flot sanguin.

A titre d'exemple, pour une valve mitrale, de 32 mm de diamètre, le diamètre total des deux plateaux est de l'ordre de 29 mm, la largeur du plus grand plateau étant sensiblement égale à la valeur du rayon augmentée de 10 à 15%. Ceci donne une valeur de 16,24 mm pour le volet (5) et de 12,76 mm pour le volet (6). Le décalage entre les deux axes (7) et (8) est de l'ordre de 2,5 mm, et la zone courbe de chaque volet s'étend sur environ 6 mm.

Il faut noter que la collerette (3) est conformée sur sa face interne pour former un épaulement (10) sur lequel vient en appui chaque volet (5, 6) en position de fermeture de la valve.

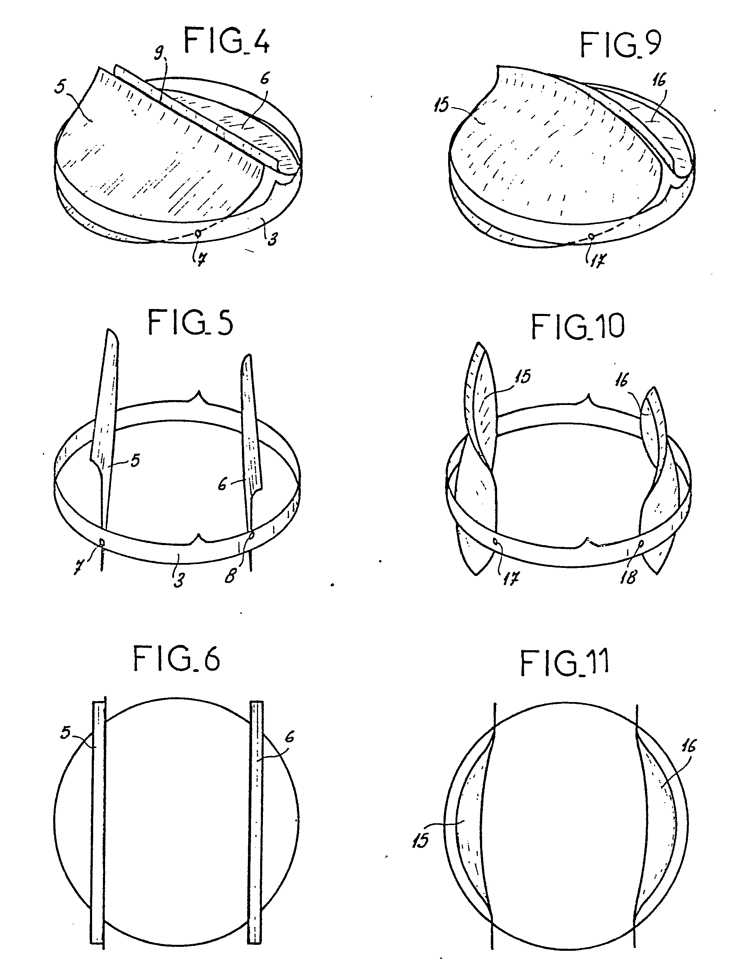

D'un point de vue pratique, lors de l'arrivée du flot sanguin, le volet (5) de plus grande surface et dont l'axe d'articulation (7) est disposé en amont de l'axe (8) du volet (6) va s'ouvrir de premier. Le régime d'écoulement turbulent provoqué par l'ouverture de ce premier volet va agir sur la partie courbée du second volet (6) pour provoquer l'ouverture spontanée de ce dernier, jusqu'à venir dans la position représentée aux figures 5 et 6.

Cette ouverture des deux volets étant obtenue très rapidement, limite au maximum la durée de perturbation d'écoulement.

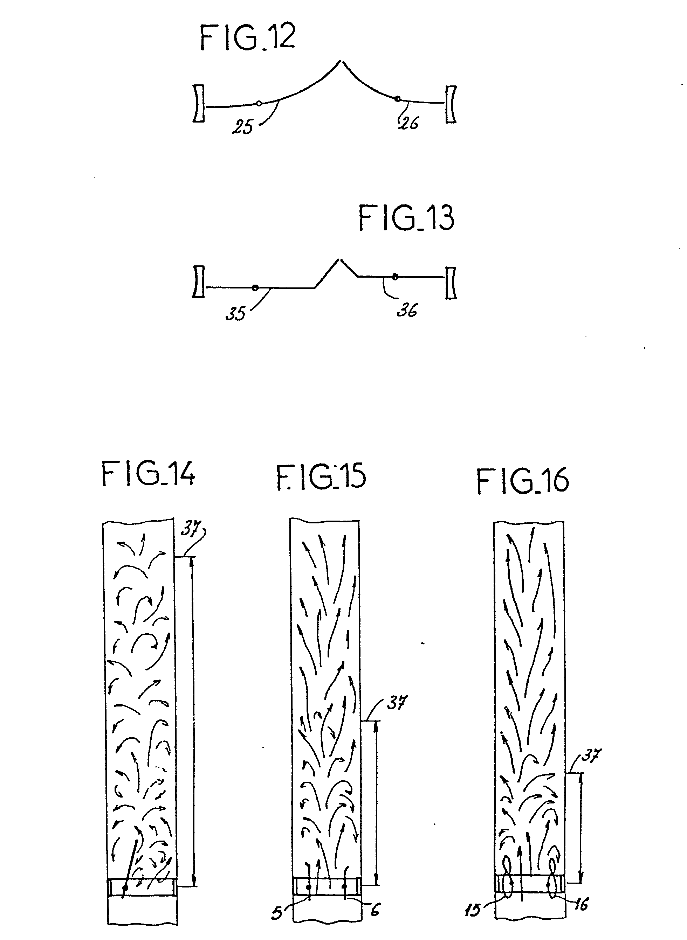

Dans la forme d'exécution représentée aux figures 7 à 11, chaque volet, respestivement (15) et (16), présente, en outre, une courbure perpendiculairement aux axes de rotation (17, 18), dans le sens d'écoulement du flot sanguin.

Comme montré au dessin et notamment à la figure 11, cet agencement assure, en position ouverte des volets (15) et (16), une proximité entre ceux-ci et la paroi dans laquelle est montée la prothèse, ce qui évite les écoulements parasites, constituant un avantage certain dans le cas de prothèses pour valves aortiques.

Les figures 12 et 13 représentent schématiquement deux autres types de valves, dans lesquelles les volets (25) et (26) sont cintrés sur la presque totalité de leur longueur dans la prothèse de la figure 12, et dans laquelle les volets (35) et (36) sont réalisés à partir de surfaces planes dans la prothèse représentée à la figure 13.

Les figures 14 à 16 représentent trois tubes équipés, respectivement, d'une valve traditionnelle, d'une valve conforme à celle des figures 1 à 6, et d'une valve conforme à celle des figures 7 à 11.

La ligne transversale (37), représentée sur chaque figure, correspond à la zone à partir de laquelle l'écoulement peut être considéré comme satisfaisant. Il ressort du dessin qu'un écoulement normal est obtenu sur une distance deux fois plus courte avec la valve selon l'invention représentée à la figure 15, qu'avec la valve traditionnelle représentée à la figure 14, et que la valve représentée à la figure 16 permet d'obtenir encore de meilleurs résultats, ceci compte tenu de la proximité entre les volets et les parois extérieures en position ouverte de la prothèse.

**Revendications**

1. Prothèse valvulaire cardiaque, du type comprenant une collerette (3) ménageant un passage susceptible d'être obturé par un disque formé de deux volets (5, 6; 15, 16) articulés sur elle et comprenant deux bords venant en contact l'un de l'autre dans la region centrale du passage, en position fermée de celui-ci, chaque volet étant articulé sur la collerette autour d'un axe (7, 8; 17, 18) situé à proximité de son extrémité la plus éloignée de son bord de contact avec l'autre volet, caractérisée en ce que les deux volets (5, 6; 15, 16) sont de surfaces inégales.

2. Prothèse selon la revendication 1, caractérisée en ce que les axes d'articulation (7, 8; 17, 18) des deux volets (5, 6; 15, 16) sont décalés l'un par rapport à l'autre dans le sens d'écoulement du flot sanguin, l'axe (7, 17) du volet (5, 15) de plus grande surface étant situé en amont de l'axe (8, 18) de l'autre volet (6, 16).

3. Prothèse selon l'une quelconque des revendications 1 et 2, caractérisée en ce que les extrémités des deux volets (5, 6; 15, 16), situées du côté du centre du disque et venant en contact pour réaliser la fermeture de la valve, sont courbées parallèlement aux axes de rotation des volets, dans la direction d'écoulement du flot sanguin.

4. Prothèse selon l'une quelconque des revendications 1 à 3, caractérisée en ce que chaque volet (5, 6; 15, 16) présente une courbure perpendiculairement à son axe de rotation dans la direction d'écoulement du flot sanguin.

5. Prothèse selon l'une quelconque des revendications 1 à 4, caractérisée en ce que la collerette (3) présente un épaulement (10) formant siège pour les volets (5, 6; 15, 16), lorsque ceux-ci sont en position fermée, ce qui procure une excellente étanchéité en absence d'écoulement du flot sanguin.

## Patentansprüche

1. Künstliche Herzklappe mit einem Kragen (3), der einen Durchgang bildet, der von einer aus zwei Klappen (5, 6; 15, 16) bestehenden Scheibe verschliessbar ist, wobei die Klappen (5, 6; 15, 16) an dem Kragen (3) angelenkt sind und zwei Ränder besitzen, die in Schliessstellung im zentralen Bereich des Durchgangs miteinander in Berührung kommen, und wobei jede Klappe an dem Kragen (3) um eine Achse (7, 8; 17, 18) gelenkig bewegbar ist, die in der Nähe desjenigen ihrer Endbereiche liegt, der von der jeweils anderen Klappe in Berührung tretenden Rand am weitesten entfernt ist, dadurch gekennzeichnet, dass die beiden Klappen (5, 6; 15, 16) ungleiche Oberflächen haben.

2. Künstliche Herzklappe nach Anspruch 1, dadurch gekennzeichnet, dass die Gelenkachsen (7 8; 17, 18) der beiden Klappen (5, 6; 15, 16) in Richtung der Blutströmung gegeneinander versetzt sind, wobei die Achse (7, 17) der Klappe (5) mit der grösseren Oberfläche stromaufwärts der Achse (8, 18) der anderen Klappe (6, 16) liegt.

3. Künstliche Herzklappe nach einem der Ansprüche 1 oder 2, dadurch gekennzeichnet, dass die Endbereiche der beiden Klappen (5, 6; 15, 16), die auf der Seite des Zentrums der Scheibe liegen und die zum Schliessen der Herzklappe miteinander in Berührung treten, parallel zu der Gelenkachse der Klappen in Richtung der Blutströmung gekrümmt sind.

4. Künstliche Herzklappe nach einem der Ansprüche 1 bis 3, dadurch gekennzeichnet, dass jede Klappe (5, 6; 15, 16) eine Wölbung senkrecht zu ihrer Gelenkachse in Richtung der Blutströmung besitzt.

5. Künstliche Herzklappe nach einem der Ansprüche 1 bis 4, dadurch gekennzeichnet, dass der Kragen (3) eine Schulter (10) besitzt, die einen Sitz für die Klappen (5, 6; 15, 16) bildet, wenn diese sich in Schliessstellung befinden, wodurch eine hervorragende Dichtigkeit bewirkt wird, wenn keine Blutströmung vorhanden ist.

## Claims

1. Valvular cardiac prosthesis of the type comprising a ring (3) forming a passage which can be closed by a disc including two valves (5, 6; 15, 16) articulated to the ring and comprising two edges coming into contact with each other in the central region of the passage in the closed position of this passage, each valve being articulated on top of the ring about an axis (7, 8; 17, 18) situated near its end which is most distant from its edge of contact with the other valve, characterised in that the two valves (5, 6; 15, 16) have surfaces of unequal size.

2. Prosthesis according to claim 1, characterised in that the axes of articulation (7, 8; 17, 18) of the two valves (5, 6; 15, 16) are moved forward or back one in relation on the other in the direction of the flow of blood, the axis (7, 17) of the valve with the larger surface (5, 15) being situated above the axis (8, 18) of the other valve (6, 16).

3. Prosthesis according to one or other of claims 1 and 2, characterised in that the ends of the two valves (5, 6; 15, 16) situated at the side of the centre of the disc and coming into contact to bring about the closing of the valve, are curved parallel to the axes of rotation of the valves in the direction of flow of the bloodstream.

4. Prosthesis according to one or other of claims 1 to 3, characterised in that each valve (5, 6; 15, 16) displays curvature perpendicular to its axis of rotation in the direction of flow of the bloodstream.

5. Prosthesis according to one or other of claims 1 to 4, characterised in that the ring (3) presents a support (10) forming a centre for the valves (5, 6; 15, 16) when these are in a closed position, which gives water- and air-tightness in the absence of flow of the bloodstream.

FIG_1

FIG_2

FIG_7

FIG_3

FIG_8

FIG_4

FIG_9

FIG_5

FIG_10

FIG_6

FIG_11

## FIG.12

25    26

## FIG.13

35    36

## FIG.14

37

## FIG.15

37

5    6

## FIG.16

37

15    16